# EUROPÄISCHE PATENTSCHRIFT

(11) **EP 2 264 168 B1**
(45) Veröffentlichungstag und Bekanntmachung des Hinweises auf die Patenterteilung: **17.12.2014**
(21) Anmeldenummer: 09007990.6
(22) Anmeldetag: 18.06.2009
(51) Int. Cl.: C12N 15/10

(54) **Verfahren zur Isolierung von Nukleinsäuren**
Method for isolating nucleic acids
Procédé d'isolation d'acides nucléiques

(43) Veröffentlichungstag der Anmeldung: 22.12.2010
(60) Teilanmeldung: 13156068.2
(73) Patentinhaber: Qiagen GmbH, 40724 Hilden (DE)
(72) Erfinder: Horlitz, Martin Dr., 40474 Düsseldorf (DE); Sprenger-Haussels, Markus Dr., 40822 Mettmann (DE)
(74) Vertreter: Roth, Carla

(56) Entgegenhaltungen:
- WO-A-2004/108925
- WO-A-2005/012523
- US-A1- 2005 009 045
- US-A1- 2005 032 105
- US-A1- 2007 072 229
- US-A1- 2007 202 511

## Beschreibung

Die vorliegende Erfindung betrifft ein Verfahren zur Isolierung und/oder Aufreinigung von Nukleinsäuren, insbesondere von extrazellulären Nukleinsäuren wie bspw. miRNA aus nukleinsäurehaltigen Proben, insbesondere Blut.

Die Isolierung von Nukleinsäuren wie DNA und RNA aus pflanzlichen, tierischen oder menschlichen Materialien sowie aus Zellkulturen oder Viruskulturen erfolgt in der Regel nach einem einheitlichen Grundmuster: die Nukleinsäuren enthaltenen Ausgangsmaterialien werden zunächst - teilweise unter Verwendung von proteinabbauenden Enzymen - aufgeschlossen. In nachfolgenden Schritten können die einzelnen Bestandteile unter Verwendung verschiedenster Methoden abgetrennt werden. Darüber hinaus können Nukleinsäuren aus Probenmaterialien isoliert werden, in denen sie frei, d.h. nicht in Zellen, vorliegen. So können freie Nukleinsäuren in künstlichen Probenmischungen vorkommen, jedoch aber auch in natürlichen Proben wie bspw. Blut. Solche frei zirkulierenden Nukleinsäuren werden auch extrazelluläre Nukleinsäuren genannt.

Die Isolierung extrazellulärer Nukleinsäuren, insbesondere kurzkettiger microRNAs (miRNAs) aus Körperflüssigkeiten wie Blut, Plasma, Serum, Liquor (CSF) oder sogar Urin ist von besonderem Interesse, da diese als Biomarker für die Diagnose von Krebs oder anderen Krankheiten dienen können. Darüber hinaus ist ihre Untersuchung von wissenschaftlichem Interesse. Ihre Analyse setzt jedoch eine effiziente Aufreinigung dieser Nukleinsäuren voraus, was durchaus eine Herausforderung ist.

US 2007/202511 offenbart ein Verfahren, bei dem kurzkettige Nukleinsäuren in Gegenwart eines chaotropen Agens, eines Alkohols in einer Konzentration von über 40% sowie einem nicht-ionischen Detergenz an eine Silikasäule gebunden und im Anschluss eluiert werden.

WO2005/12523 beschreibt ein Verfahren zur Isolierung kleiner RNA Moleküle wie miRNAs, bei dem die Zellen mit einer Lyselösung aufgeschlossen und eine Alkohol-Lösung zum Zelllysat gegeben wird, das Lysat mit einem festen Träger in Kontakt gebracht wird und die gebundene RNA im Anschluss von dem festen Träger eluiert wird. Die Lyselösung kann ein chaotropes Agens und ein Detergenz enthalten.

US2007/072229 offenbart ein Verfahren zur Isolierung von RNA, darunter auch miRNA. Es wird eine sanfte Lyse durchgeführt, so dass zwar die Zellmembran zerstört und die RNA freigesetzt wird, die Membran des Zellnukleus jedoch intakt bleibt. Das so erhaltene Lysat wird ohne Aufreinigung der RNA weiterverarbeitet.

US2005/032105 beschreibt ein Verfahren zur Isolierung von DNA unter Einsatz von Lithiumsalzen, wobei chaotrope Agenzien vermieden werden sollen.

US2005/009045 offenbart ein Verfahren zur Isolierung von Nukleinsäuren. Die Probe wird durch Zugabe eines kationischen Detergenz, einer Protease und einem Puffer lysiert und die freigesetzte Nukleinsäure wird im Anschluss isoliert.

WO2004/108925 offenbart ein Verfahren zur sequenziellen Isolierung von DNA und RNA aus der gleichen Probe, bei dem chaotropen Agenzien und Alkohol eingesetzt wird.

Im Stand der Technik bekannte Verfahren sind zwar in der Lage, kurze RNAs aus Körperflüssigkeiten wie Vollblut und auch zellfreien Blutfraktionen (Plasma oder Serum) aufzureinigen, jedoch nur mit vergleichweise geringer Ausbeute. Darüber hinaus fehlt es an Verfahren, um insbesondere frei zirkulierende Nukleinsäuren wie bspw. microRNAs (miRNAs) aus Plasma oder Serum effizient aufzureinigen. Frei zirkulierende microRNAs oder auch fötale bzw. Placenta-spezifische Nukleinsäuren aus Plasma oder Serum stellen jedoch wichtige Biomarker für die molekulare Diagnostik dar, z.B. für die Diagnose von Tumoren oder für Schwangerschafts- und nicht-invasive Pränataldiagnostik.

Es besteht daher ein Bedarf an verbesserten Verfahren zur Isolierung von kurzkettigen Nukleinsäuren wie miRNAs aus Körperproben wie Blut bzw. Plasma oder Serum, die die sehr gering konzentrierte und oftmals auch sehr kurze Nukleinsäuren (≤ 50 nt) enthalten. Neben einer effizienten Aufreinigung sollen vorzugsweise auch große Probenvolumina verarbeitet werden können. Ein solches Verfahren würde es ermöglichen, sehr gering konzentrierte Nukleinsäuren wie bspw. miRNAs, die z.B. aus einem kleinen Tumorgewebestück oder aus der Plazenta ins Blut (bzw. Plasma) entlassen werden, mit hoher Empfindlichkeit nachweisen zu können.

Der vorliegenden Erfindung liegt daher die Aufgabe zugrunde, ein Verfahren zur Isolierung und/oder Aufreinigung von Nukleinsäuren zur Verfügung zu stellen, mit dem sich insbesondere extrazelluläre und auch kurzkettige Nukleinsäuren wie insbesondere miRNA aus Proben effizient isolieren lassen.

Die Aufgabe wird vorliegend gelöst durch ein Verfahren zur Isolierung und/oder Aufreinigung von Nukleinsäuren, insbesondere frei zirkulierenden Nukleinsäuren, aus einem nukleinsäurehaltigen Ausgangsmaterial, das durch die folgenden Verfahrensschritte gekennzeichnet ist:
(a) Bindung der Nukleinsäuren an ein nukleinsäurebindendes Trägermaterial, indem man das Ausgangsmaterial in Gegenwart wenigstens einer chaotropen Verbindung, wenigstens eines anionischen und wenigstens eines nicht-ionischen Detergenz und wenigstens eines Alkohols ausgewählt aus Isopropanol und Ethanol, mit dem nukleinsäurebindenden Trägermaterial in Kontakt bringt, wobei der Alkohol in einer Konzentration ≥ 40 % (v/v) vorliegt und wobei das nukleinsäurebindende Trägermaterial eine nukleinsäurebindende Membran, eine lose Schüttung eines porösen oder nicht porösen Trägermaterials, ein nukleinsäurebindender Filter oder eine nukleinsäurebindende Filterschicht ist;
(b) Elution der gebundenen Nukleinsäuren von dem nukleinsäurebindenden Trägermaterial.

Durch die erfindungsgemäß optimierte Extraktionschemie werden Nukleinsäuren, insbesondere frei zirkulierende Nukleinsäuren wie insbesondere kurzkettige RNAs und insbesondere microRNAs (miRNAs), effizient an das nukleinsäurebindende Trägermaterial gebunden. Dies wird erreicht durch eine hohe Alkoholkonzentration von ≥ 40 % (v/v) während der Bindung bei gleichzeitigem Vorliegen von einer chaotropen Verbindung und wenigstens zwei Detergenzien, nämlich wenigstens eines anionischen und wenigstens eines nicht-ionischen Detergenz. Bei diesen Bedingungen ist die Ausbeute an kurzkettigen Nukleinsäuren, insbesondere miRNA, deutlich höher und damit effizienter als mit einem Standardprotokoll gemäß dem Stand der Technik. Diese vorteilhafte Erhöhung der Ausbeute wird insbesondere auch aufgrund des Einsatzes von zwei verschiedenen Detergenzien ermöglicht, was überraschend war. Zudem ermöglicht die erfindungsgemäße Anwendung einer Kombination von zwei Detergenzien das Passieren der Probenflüssigkeit über kleinflächige nukleinsäurebindende Träger wie bspw. Membrane, vorzugsweise Silikamembrane, ohne dass diese verstopfen. Dadurch ermöglicht es das erfindungsgemäße Verfahren in vorteilhafter Weise, große Probenvolumina auf einer kleinflächigen Membran zu verarbeiten und somit einen hohen Konzentrationseffekt hinsichtlich der zu isolierenden Nukleinsäuren zu erzielen.

Das Trägermaterial kann ausgewählt sein aus der Gruppe der Silikahaltigen Materialien, Silikagel, Siliziumdioxid, Glas, Zeolith, Aluminiumoxid, Titandioxid, Zirkoniumdioxid, Kaolin, Kieselalgen, Kieselgel, oder Keramik. Das poröse oder nicht poröse Trägermaterial kann in Form loser Schüttungen vorliegen oder als Filterschichten oder Membrane ausgebildet sein.

Gemäß einer bevorzugten Ausführungsform handelt es sich bei dem nukleinsäurebindenden Trägermaterial daher um eine nukleinsäurebindende Membran oder einen nukleinsäurebindenen Filter bzw. eine Filterschicht, die vorzugsweise feinporig ist. Diese können jede beliebige Form aufweisen und so bspw. rund oder eckig sein. Der Durchmesser der Membrane und/oder Filter bzw. Filterschichten ist gemäß einer Ausführungsform ≤ 10mm, vorzugsweise ≤ 7 mm. Die Fläche ist daher möglichst klein, um ein kleines Elutionsvolumen erzielen zu können. Gemäß einer Ausführungsform liegt diese bei ≤ 80 mm², vorzugsweise ≤ 40 mm². Die Dicke der Membran bzw. des Filters/Filterschicht liegt gemäß einer Ausführungsform in einem Bereich von 0,5 - 3 mm, vorzugsweise 0,7 - 1.3 mm. Das Flächengewicht liegt gemäß einer Ausführungsform bei 50 - 400 g/m², vorzugsweise bei 100 - 180 g/m². Die Partikelretention (d.h. die untere Grenze der Durchlässigkeit für partikuläre Substanzen) liegt üblicher Weise in einem Bereich von 0,05 µm - 100 µm, vorzugsweise 0,1 - 20 µm. Besonders geeignet ist der Einsatz einer entsprechenden Silikamembran.

Beim Einsatz von Membranen oder Filter bzw. Filterschichten besteht jedoch insbesondere das Problem, dass diese bei den erfindungsgemäß eingesetzten hohen Alkoholkonzentrationen oftmals verstopfen, ggf. durch eine unspezifische Präzipitation von Plasmabestandteilen. Der Einsatz der hohen Alkoholkonzentrationen erhöht daher zwar die Ausbeute an den gewünschten kurzkettigen Nukleinsäuren, erschwert jedoch die Prozessierung der Proben. Hier hat sich überraschender Weise gezeigt, dass die gleichzeitige Anwesenheit von zwei Detergenzien, vorzugsweise einem ionischen und einem nicht-ionischen Detergenz, das Solubilisieren der Präzipitate und daher auch das Vakuum- oder zentrifugationsbasierte Passieren großer Bindeansatzvolumina über eine kleinflächige Membran erlauben. Dies ist ein besonderer Vorteil bei der Verarbeitung größerer Probenvolumina (bspw. von wenigstens 1 ml, vorzugsweise wenigstens 3 ml bzw. wenigstens 4 ml). Derartige Probenvolumina sind in der Regel für die Isolation extrazellulärer Nukleinsäuren erforderlich, wie bspw. bei der Isolation der sehr kurzkettigen miRNAs aus Plasma, da dieser Nukleinsäure-Typus in der Probe regelmäßig in nur sehr geringen Konzentrationen vorliegt. Das erfindungsgemäße Verfahren ermöglicht jedoch in vorteilhafter Weise das effektive Verarbeiten großer Probenvolumina und damit die effektive Isolierung der frei zirkulierenden Nukleinsäuren aufgrund der besonderen Bindungsbedingungen mit einer guten Ausbeute.

Es wird wenigstens ein ionisches Detergenz in Kombination mit wenigstens einem nicht-ionischen Detergenz eingesetzt, wobei das ionische Detergenz ein anionisches Detergenz ist. Diese Kombination hat sich als vorteilhaft erwiesen. Das ionische Detergenz wird vorzugsweise in einer Menge von wenigstens ≥ 0,05 % (w/v) eingesetzt, besonders geeignet hat sich ein Bereich von 0,1 bis 5% (w/v), vorzugsweise 0,1 bis 2 % (w/v), besonders bevorzugt 0,1 bis 0,2 % (w/v) erwiesen. Das nicht-ionische Detergenz wird gemäß einer Ausführungsform in höheren Mengen von wenigstens ≥ 0,5% (w/v), vorzugsweise ≥ 1 % (w/v), besonders bevorzugt ≥ 4 % (w/v) eingesetzt. Besonders geeignet haben sich Konzentrationen in einem Bereich von wenigstens 5 bis 8 % (w/v) erwiesen.

Ionische Detergenzien umfassen anionische, kationische und zwitterionische Detergenzien. Gemäß Anspruch 1 werden anionische Detergenzien eingesetzt. Vorzugsweise sind die erfindungsgemäß eingesetzten Detergenzien Tenside.

Bevorzugte anionische Detergenzien sind Sulfate, Sulfonate, Phosphate und Carbonsäuren, die vorzugsweise als Salz, bspw. als Alkalisalz oder Erdalkalisalze z.B. als Natrium-, Lithium- oder Kaliumsalz, oder als freie Säure vorliegen. Anionische Detergenzien können insbesondere Sulfate von Fettalkoholen, insbesondere mit einer unverzweigten oder verzweigten Alkylkette von 4 bis 28 Kohlenstoffatomen, bevorzugt 8 bis 18 Kohlenstoffatomen, oder Alkylarylsufonate, insbesondere lineare Alkylbenzolsulfonate sein. Spezifische Beispiele anionischer Detergenzien sind Natriumdodecylsulfat (SDS), Lithiumdodecylsulfat, Natriumoctylsulfat, Natriumdodecylsulfonat, Natriumdecylsulfonat, Natriumoctylsulfonat, Dodecylbenzolsulfonsäure (DDBSA), N-Lauroylsarcosin, Natriumcholat, Natriumdesoxycholat. Beispiele für kationische Detergenzien sind insbesondere quaternäre Ammoniumverbindungen wie Cetyltrimethylammoniumbromid (CTAB). Zwitterionische Detergenzien sind vorzugsweise 3-[(3-Cholamidopropyl)dimethylammonio]-1-propanesulfonate (CHAPS) oder verwandte Detergenzien.

Quaternäre Ammoniumverbindungen mit mindestens einer langen Alkylkette können als kationische Detergenzien eingesetzt werden.

Nicht-ionische Detergenzien umfassen Polyoxyethylen-Detergenzien, insbesondere Polyoxyethylen-Alkylether, wobei der Alkylanteil bevorzugt eine unverzweigte oder verzweigte Alkylgruppe aus 4 bis 28, vorzugsweise 8 bis 24, mehr bevorzugt 12 bis 20 Kohlenstoffatomen ist und der Polyoxyethylenanteil mindestens 2, vorzugsweise mindestens 4, mehr bevorzugt 6 bis 24 Ethyleneinheiten aufweist, Polyoxyethylen-Alkylarylether, bei denen eine mit einer Alkylgruppe vorzugsweise in para-Stellung substituierte Phenylgruppe an die Polyoxyethylengruppe gekoppelt ist, und Detergenzien der Triton X-Serie. Spezifische Beispiele für geeignete nicht-ionische Detergenzien sind Brij 58, Brij 30, Brij 35, Brij 56, Brij 72, Brij 90, Igepal CA-630, Span 20, Span 60, Span 80, Tween 20, Tween 80, Triton X-100, Triton X-114, Triton X-450, Digitonin und Nonidet P-40.

Geeignete Detergenzien für die vorliegende Erfindung sind unter anderem auch im Produktkatalog der Firma Sigma-Aldrich (Life Science, Biochemicals & Reagents) zu finden.

Besonders geeignet hat sich eine Kombination von Brij 58 und SDS erwiesen.

Gemäß Anspruch 1 ist der Alkohol ausgewählt aus Ethanol und Isopropanol. Auch Mischungen der entsprechenden Alkohole können eingesetzt werden. Besonders bevorzugt wird Isopropanol oder ein Alkohol bzw. Alkoholgemisch mit Isopropanol ähnlichen Eigenschaften eingesetzt. Die Alkoholkonzentration während der Bindung liegt bei ≥ 40% (v/v), vorzugsweise ≥ 42,5% (v/v). Je nach Alkohol können aber auch höhere Alkoholkonzentrationen von ≥ 50 % oder sogar ≥ 60 % eingesetzt werden. Die Alkoholkonzentration liegt jedoch vorzugsweise bei ≤ 80% (v/v), besonders bevorzugt ≤ 70% (v/v).

Geeignete chaotrope Verbindungen, die die Bindung der frei zirkulierenden Nukleinsäuren an das Trägermaterial fördern, können auf Basis der sogenannten Hofmeister-Reihe ausgewählt werden. Danach sind stark chaotrope Anionen NO₃⁻, ClO₄⁻, SCN⁻, NCS⁻, und Cl₃CCOO⁻. Stark chaotrope Kationen sind bspw. Ba²⁺ und Guanidinium. Vorzugsweise werden als chaotrope Verbindungen Thiocyanate, Isothiocyanate und/oder Perchlorate eingesetzt, insbesondere Guanidiniumthiocyanat oder Guanidiniumisothiocyanat. Weitere chaotrope Agenzien, die erfindungsgemäß eingesetzt werden können sind Guanidiniumhydrochlorid, Guanidiniumcarbonat, Natriumiodid, Natriumperchlorat, Harnstoff und Thioharnstoff. Wie ausgeführt können auch Mischungen mehrere chaotrope Agenzien zum Einsatz kommen.

Vorzugsweise wird die chaotrope Verbindung in hohen Konzentrationen eingesetzt. Vorzugsweise liegt die Konzentration bei wenigstens ≥ 1 mol/l. Geeignete Bereiche liegen daher zwischen ≥ 1 und ≤ 8 mol/l, vorzugsweise in einem Bereich von ≥ 1,3 bis ≤ 5, ≥ 1,3 bis ≤ 4 und besonders bevorzugt in einem Bereich von ≥ 1,0 bis ≤ 3 mol/l.

Verschiedene Materialien und insbesondere biologische Materialen können als nukleinsäurehaltige Ausgangsmaterialien zum Einsatz kommen. Der Bergriff "Ausgangsmaterial" umfasst jegliches nukleinsäurehaltige Material und damit sowohl natürliche Proben, als auch aufbereitete Proben bzw. Probenbestandteile und ferner künstliche Proben wie bspw. Lösungen enthaltend Nukleinsäuren (bspw. aus einer Synthese oder PCR-Reaktion). Besonders eignet sich das Verfahren zur Isolierung/Aufreinigung von freien Nukleinsäuren aus nicht-zellhaltigen Proben bzw. entsprechend aufbereiteten Proben bzw. Probenbestandteilen. Um eine zellfreie Probe zu erhalten, können die Zellen bspw. durch Zentrifugation abgetrennt werden. Insbesondere eignet sich das erfindungsgemäße Verfahren bspw. für die Isolierung von Nukleinsäuren wie DNA und/oder RNA aus Probenmaterialen humanen oder tierischen Ursprungs, insbesondere klinischen Proben, wie Blut, Plasma, Serum, Mundspülflüssigkeit, Urin, Zerebralflüssigkeit, Liquor, Sputum, Stuhl, Punktate, Epithelabstriche, Biopsien und anderen Geweben oder Knochenmarkproben. Insbesondere ist das erfindungsgemäße Verfahren zur Isolierung frei zirkulierender Nukleinsäuren wie beispielsweise Tumor-DNA und -RNA, fötaler Nukleinsäuren sowie miRNAs aus Körperflüssigkeiten wie Blut, insbesondere Plasma und/oder Serum geeignet.

Nukleinsäuren, die mit dem vorliegenden Verfahren isoliert werden können, sind bspw. DNA, RNA, mRNA, mitochondriale, modifizierte Nukleinsäuren, bspw. epigenetisch modifizierte, einzelsträngige, doppelsträngige, zirkuläre, Plasmid, Cosmid, artifizielle oder synthetische Nukleinsäuren, sowie cDNA und Fragmente davon. Das erfindungsgemäße Verfahren eignet sich insbesondere zur Anreicherung von kurzkettigen Nukleinsäuren (bspw. DNA und RNA in jeglicher Form, einschließlich nicht kodierender RNA wie bspw. miRNA oder synthetische Nukleinsäuren) mit einer Länge von ≤ 1000nt, ≤ 800nt, ≤ 500 nt, ≤ 300 nt, ≤ 200 nt, ≤ 100 nt, oder ≤ 50 nt.. Bevorzugt wird DNA und/oder RNA aufgereinigt. Besonders effizient lässt sich DNA oder RNA einer Länge ≤ 50 Nukleotiden aufreinigen. Mit dem erfindungsgemäßen Verfahren wird je nach nukleinsäurehaltigem Ausgangsmaterial in der Regel ein Nukleinsäuregemisch enthalten, in dem insbesondere auch die kurzkettigen Nukleinsäuren, enthalten sind. Diese können dann weiterverarbeitet werden, bspw. prozessiert, modifiziert oder analysiert werden. Zur Isolierung von RNA wird die aufgereinigte Nukleinsäure vorzugsweise mit DNase behandelt. In der so enthaltenden RNA ist bei Einsatz des erfindungsgemäßen Verfahrens auch die kurzkettige RNA enthalten.

Die aufzureinigenden Nukleinsäuren weisen vorzugsweise eines oder mehrere der nachfolgenden Merkmale auf:
a) sie weisen eine Länge von ≤ 500nt, ≤ 400nt und/oder ≤ 300 nt und/oder ≤ 100nt und oder ≤ 50nt auf;
b) es handelt sich um RNA und/oder DNA;
c) es handelt sich um extrazelluläre Nukleinsäuren;
d) es handelt sich um nicht-proteinkodierende RNAs;
e) es handelt sich um RNAi auslösende Nukleinsäuren, insbesondere siNAs.

Der Begriff "siNA" (short interfering nucleic acid) bezieht sich insbesondere auf Nukleinsäuren oder funktionale Varianten oder Derivative davon, die in der Lage sind, RNAi (RNA interference) Prozesse auszulösen. Diese können sowohl einzelsträngig als auch doppelsträngig vorliegen. Gemäß einer Ausführungsform ist die siNA ein doppelsträngiges Polynukleotid, das selbst komplementäre sense und antisense Stränge aufweist. Auch Vorläufer von entsprechenden doppelsträngigen Molekülen sind von dem Begriff umfasst. Insbesondere seien ferner micro-RNAs (miRNAs) als siNAs erwähnt. Es hat sich herausgestellt, dass micro-RNAs sowohl für wissenschaftliche als auch für diagnostische Zwecke wertvoll sind (Nutzung der miRNAs als Biomarker). Micro-RNAs sind kurze, hoch konservierte, nicht kodierende RNA Moleküle, die eine wichtige Rolle in dem komplexen Netzwerk der Genregulation insbesondere beim Gen-Silencing (Stilllegung von Genen) spielen. Micro-RNAs regulieren die Genexpression hoch spezifisch auf post-transkriptionalem Level. Es wurde gefunden, dass diese auch frei in Körperflüssigkeiten, wie insbesondere Blut vorliegen und daher wichtige diagnostische Marker darstellen können. Entsprechend besteht ein Bedarf, miRNAs mit großer Effizienz aus Körperflüssigkeiten, wie insbesondere Blut aufzureinigen. miRNAs weisen jedoch im Allgemeinen eine Größe von weniger als 25nt auf und liegen auch nur in geringen Konzentrationen vor, was ihre Aufreinigung erschwert.

Da die kurzkettigen Nukleinsäuren, wie insbesondere miRNA regelmäßig nur in geringen Konzentrationen in den Proben vorliegen, ist es wünschenswert ein großes Probenvolumen zu verarbeiten. Daher liegt das nukleinsäurehaltige Ausgangsmaterial gemäß einer Ausführungsform in einem Volumen ≥ 1 ml, vorzugsweise ≥ 3 ml vor.

Um eine ausreichende Konzentrierung der Nukleinsäuren zu erzielen, wird ferner vorzugsweise ein kleines Elutionsvolumen eingesetzt. Dieses liegt vorzugsweise in einem Bereich von 10 bis 200 µl, vorzugsweise 10 bis 150 µl. Dies lässt sich insbesondere bei Einsatz einer kleinflächigen nukleinsäurebindenden Membran, vorzugsweise einer Silikamembran oder eines entsprechenden Filters bzw. Filterschicht erzielen.

Mit der Erfindung wird daher ein optimiertes Protokoll insbesondere zur Aufreinigung von kurzkettigen Nukleinsäuren, insbesondere extrazellulären Nukleinsäuren und insbesondere miRNA zur Verfügung gestellt, welches zum einen das Prozessieren eines großen Probenvolumens erlaubt, um dadurch die absolute Ausbeute an Nukleinsäuren und insbesondere miRNA, zu erhöhen. Der Vollständigkeit halber sei darauf hingewiesen, dass neben den kurzkettigen Nukleinsäuren, wie insbesondere den miRNAs auch andere Nukleinsäuren (lang- und kurzkettige) mit aufgereinigt werden können. Dies hängt auch von der Art der Probe ab und welche Nukleinsäuren darin enthalten sind. Entscheidend ist lediglich, dass die gewünschten Nukleinsäuren, wie insbesondere kurzkettige Nukleinsäuren wie miRNAs in einer ausreichenden Menge in der aufgereinigten Probe vorliegen. Sofern eine spezifische Anreicherung der entsprechenden kurzkettigen Nukleinsäuren (bspw. gegenüber langkettigen Nukleinsäuren) gewünscht ist, können geeignete Vorschritte durchgeführt werden, um beispielsweise längerkettige Nukleinsäuren zunächst abzureichern. Dies kann bspw. über eine Einstellung der Bindebedingungen erfolgen, bspw. durch Einsatz einer geringeren Alkoholkonzentration und/oder einer niedrigeren Konzentration einer chaotropen Verbindung. Sofern RNA spezifisch aufgereinigt werden soll, ist die Durchführung eines DNase Verdaus vorteilhaft.

Ferner erlaubt das erfindungsgemäße Verfahren die Nutzung eines kleinen Elutionsvolumen (idealerweise 20 bis 50 µl), so dass die Nukleinsäuren im Eluat in hoher Konzentration vorliegen. Durch den Einsatz einer modifizierten Bindechemie wird die Ausbeute an sehr kurzen Nukleinsäuren von unter 50 Nukleotiden und vorzugsweise unter 25 Nukleotiden deutlich gegenüber den im Stand der Technik bekannten Verfahren erhöht. Die vorliegende Erfindung liefert daher eine wertvolle Ergänzung zu den im Stand der Technik bestehenden Verfahren zur Aufreinigung entsprechender Nukleinsäuren.

In bestimmten Fällen kann die Probe ohne Vorbehandlung in dem erfindungsgemäßen Verfahren eingesetzt werden. In vielen Fällen kann es erforderlich sein, die Probe jedoch zunächst durch eine geeignete Methode aufzuschließen und das in der Probe enthaltene biologische Material freizusetzen. Verfahren zum Aufschluss von Proben und Zellen sind dem Fachmann bekannt und können chemischer, enzymatischer oder physikalischer Natur sein. Auch eine Kombination dieser Verfahren ist möglich.

Hierbei können sich verschiedene Faktoren für unterschiedliche biologische Materialien als vorteilhaft herausstellen; prinzipiell sind folgende Methoden gut geeignet: Lyse mit Hilfe von ionischen und nicht-ionogenen Detergenzien, wie z. B. SDS, LiDS oder Sarkosyl in geeigneten Puffern, der Einsatz von chaotropen Salzen, wie beispielsweise Guanidiniumhydrochlorid (GHCL), Guanidiniumthiocyanat (GTC), Guanidiniumisothiocyanat (GITC), Natriumiodid, Natriumperchlorat u. a.; mechanisches Auseinanderreißen, wie z. B. mittels einer French Press, Ultraschall, Mahlen mit Glaskugeln, Nickelkugeln, Aluminium oder in flüssigen Stickstoff; enzymatische Lyse, beispielsweise mit Lysozym, Proteinasen, Proteinase K oder Zellulasen oder mittels anderer kommerziell erhältlichen Enzyme für die Lyse; Lyse der Zellen mittels Bakteriophagen oder Virusinfektionen; Gefriertrocknen; osmotischer Schock; Mikrowellenbehandlung; Temperaturbehandlung; beispielsweise Erwärmen oder Kochen oder Gefrieren, z. B. in Trockeneis oder flüssigem Stickstoff und Auftauen; alkalische Lyse. Wie ausgeführt, stellen die vorstehenden Verfahren Stand der Technik zur Lyse da, die hinlänglich bekannt sind und daher nicht im Detail erläutert werden müssen.

Bei der Aufreinigung von frei zirkulierenden Nukleinsäuren und insbesondere RNAi auslösenden Nukleinsäuren wie bspw. miRNAs aus einer Blutprobe, können zunächst die Zellen und weitere feste Bestandteile des Blutes abgetrennt (bspw. durch Zentrifugation) und das so gewonnene Plasma weiterverarbeitet werden. Das Plasma ist üblicherweise frei von Zellen, enthält jedoch die extrazellulär vorliegenden Nukleinsäuren, bspw. fötale Nukleinsäuren, Tumor DNA und/oder RNA und miRNAs. Bei der Aufreinigung von freien Nukleinsäuren wie bspw. miRNA aus Plasma ist keine eigentliche Zelllyse erforderlich, um die Nukleinsäuren freizusetzen, da diese schon in frei zirkulierender Form vorliegen. Dies gilt auch für andere Proben, bei denen die Nukleinsäuren frei vorliegen und sich entsprechend nicht in Zellen befinden. Solche extrazellulären, d.h. frei zirkulierenden bzw. frei vorliegenden Nukleinsäuren können jedoch assoziiert mit Proteinen und/oder anderen Stoffen vorliegen. Aus diesem Grunde wird das nukleinsäurehaltige Ausgangsmaterial, beispielsweise das Blutplasma, zunächst mit einem Release-Puffer behandelt, der sicherstellt, dass die Nukleinsäuren aus der assoziierten Form freigesetzt werden. Der Release-Puffer ist in seiner Funktion und Zusammensetzung ähnlich einem Lyse-Puffer, der zum Zellaufschluss eingesetzt wird. Durch den Release-Puffer werden in der Probe geeignete Bedingungen für die Freisetzung der Nukleinsäuren erzeugt, so dass diese nicht komplexiert vorliegen. Durch die Zugabe des Release-Puffers wird die Nukleinsäure der Aufreinigung besser zugänglich. Das erfindungsgemäße Verfahren kann entsprechend auch bei zellfreien Ausgangsmaterialien eingesetzt werden.

Der entsprechende Release-Puffer kann, je nach Zusammensetzung, auch als Lyse-Puffer fungieren. In der Regel können die üblichen Lyse-Puffer auch als Release-Puffer eingesetzt werden. Die Begriffe werden hier daher auch als Synonyme benutzt.

Erfindungsgemäß ist der Einsatz von chaotrophaltigen Release - bzw. Lyse-Puffern besonders effektiv. Dies insbesondere, da auch die Zusammensetzung des Release- bzw. Lyse-Puffers die Bedingungen beeinflusst, unter denen die Nukleinsäuren an das Trägermaterial binden. Die Bindungsbedingungen in der Probe, die erfindungsgemäß entscheidend für die Effektivität des erfindungsgemäßen Verfahrens sind, können daher auch durch geeignete Wahl des Release- bzw. Lyse-Puffers bspw. in Kombination mit einem Bindepuffer eingestellt werden. Release- bzw. Lysepuffer gemäß der vorliegenden Erfindung enthalten eine chaotrope Verbindung, wie z. B. GTC oder GHCL und ggf. bereits die erfindungsgemäß eingesetzten Detergenzien, wie z. B. SDS und Brij 58. Diese Agenzien können in wässriger Lösung oder in einer Pufferlösung, d. h. als sog. Release- oder Lysepuffer vorliegen. Als Puffer kann jeder geeignete Puffer, wie beispielsweise Tris, Bicin, Tricin oder Phosphatpuffer eingesetzt werden. Alternativ kann das Lyse- bzw. Release - Agens auch getrennt zugegeben werden. Geeignete Konzentrationen und Mengen der Lyse- bzw. Release - Agenzien variieren in Abhängigkeit von den betreffenden Systemen, Art der Zellen etc. und können durch den Fachmann bestimmt werden.

Chaotrope Verbindungen sind auch während der Bindung der Nukleinsäuren an das Trägermaterial in dem Probengemisch vorhanden. Einzelheiten hierzu wurden oben im Detail erläutert. Die chaotropen Verbindungen können bspw. aus dem Lyse- bzw. Release-Puffer stammen und/oder aber separat bspw. in Form eines Bindepuffers hinzugegeben werden. Entscheidend sind letztlich die Bindungsbedingungen in der Probe während der Bindung der Nukleinsäuren an das Trägermaterial. Hier kann die chaotrope Verbindung letztlich bis zur Löslichkeitsgrenze vorliegen. Der Einsatz chaotroper Verbindungen ist vorteilhaft für die effiziente Bindung der Nukleinsäuren und insbesondere der kurzkettigen Nukleinsäuren.

Die Detergenzien können ebenfalls entweder mit dem Release/Lysepuffer zugegeben werden, oder aber bspw. Bestandteil des Bindepuffers sein. Auch ist eine separate Zugabe möglich. Detergenzien bewirken eine effiziente Solubilisierung verschiedener Bestandteile in der Probe, so bspw. von Serum- und Plasmabestandteilen. Dies ist insbesondere bei Einsatz einer Silikamembran von Vorteil. Wie sich vorliegend gezeigt hat, wurde die Effizienz des erfindungsgemäßen Aufreinigungsverfahrens durch Einsatz wenigstens zweier Detergenzien, vorzugsweise einem ionischen und einem nicht-ionischen Detergenz, in überraschender Weise deutlich verbessert.

Wie ausgeführt, können bspw. nukleinsäurebindende Filter bzw. Filterschichten und Membrane und insbesondere Silikamembrane eingesetzt werden. Flüssigkeiten können hier durch Zentrifugation oder Anlegung von Vakuum oder durch Druck oder Gravitation entfernt werden. In vielen Anwendungen des erfindungsgemäßen Verfahrens, insbesondere bei der Aufreinigung von Nukleinsäuren aus Blutproben (vorzugsweise Plasma oder Serum), werden die aufzureinigenden Nukleinsäuren in geringen Konzentrationen in großen Volumina vorliegen. Bei Einsatz einer Silikamembran besteht im Stand der Technik oft das Problem, dass große Probenvolumina die Membran verstopfen können. Nach dem erfindungsgemäßen Verfahren tritt dieses Problem jedoch nicht auf. Daher ist das Verfahren auch weniger zeitintensiv und auch automatisiert durchführbar.

Das nukleinsäurebindende Trägermaterial ist gemäß einer Ausführungsform eine nukleinsäurebindende Festphase aus der Gruppe der silikahaltigen Materialien, Silikagel, Siliziumdioxid, Glas, Zeolith, Aluminiumoxid, Titandioxid, Zirkoniumdioxid, Kaolin, Kieselgel, Keramik oder polymere Trägermaterialien sowie Polystyrolkügelchen oder beschichtete magnetische oder paramagnetische Partikel. Entscheidend ist letztlich, dass das Trägermaterial in der Lage ist, Nukleinsäuren zu binden. Besonders bevorzugt werden Silikamaterialien eingesetzt. Hier haben sich sowohl der Einsatz von Silikamembranen oder Silikafilter bzw. Filterschichten als auch von magnetischen (bspw. super-, para-, ferri- oder ferromagnetischen) Partikeln bewährt, die eine Silika- oder Glasoberfläche aufweisen. Diese können im Wesentlichen perl- oder kugelförmig sein und weisen vorzugsweise eine Partikelgröße im Bereich von 0,02-30 µm, vorzugsweise 0,05-15 µm und besonders bevorzugt von 0,1-10 µm auf. Magnetische Silikapartikel, die vorteilhafter Weise im erfindungsgemäßen Verfahren eingesetzt werden können, sind z. B. in der internationalen Patentanmeldung WO 01/71732 beschrieben, worauf hiermit voll inhaltlich Bezug genommen wird. Wie ausgeführt wird vorzugsweise eine nukleinsäurebindende Membran oder ein nukleinsäurebindender Filter eingesetzt.

Optional können bei dem erfindungsgemäßen Verfahren Waschschritte mit im Stand der Technik bekannten Waschpuffern und/oder Waschlösungen durchgeführt werden. Die Waschpuffer enthalten gemäß einer Ausführungsform chaotrope Substanzen. Vorzugsweise enthalten die Waschpuffer wenigstens 40% (v/v), vorzugsweise wenigstens 50% (v/v) eines oder mehrerer Alkohole, um die Bindung der kurzkettigen Nukleinsäuren an die feste Phase zu erhalten und somit ein versehentliches Auswaschen der Nukleinsäuren von dem Träger zu verhindern. Ein Waschpuffer enthaltend chaotrope Substanzen und wenigstens 50% (v/v) eines oder mehrerer Alkohole ist besonders bevorzugt.

Die an das Trägermaterial gebundenen Nukleinsäuren werden dann vom Trägermaterial abgetrennt, bspw. in an sich bekannter Weise eluiert, sofern eine Gewinnung der kurzkettigen Nukleinsäuren gewünscht ist. Geeignete Elutionspuffer und Lösungen sind im Stand der Technik bekannt.

Die erfindungsgemäß isolierten Nukleinsäuren können dann in bekannter Weise weiterverarbeitet, also bspw. analysiert werden. Ferner kann das Verfahren dazu eingesetzt werden, Nukleinsäuren aus einer Probe zu entfernen.

Das erfindungsgemäße Verfahren ist insbesondere für die Anwendung in der Diagnostik geeignet. Das erfindungsgemäße Verfahren kann manuell oder aber auch automatisiert durchgeführt werden und ist daher auch zur Anwendung auf entsprechenden Aufreinigungsrobotern einsetzbar. Weitere Anwendungsbereiche finden sich beispielsweise in der Forensik und in anderen Bereichen, wo die Aufreinigung kleiner Nukleinsäuren wie bspw. miRNA entscheidend ist.

Mit der Erfindung wird ferner konkret ein Verfahren zur Isolierung und/oder Anreicherung von RNAi auslösenden Nukleinsäuren, insbesondere miRNAs aus einer Blutprobe, insbesondere aus Blutplasma, zur Verfügung gestellt, welches durch die oben beschriebenen Merkmale gekennzeichnet ist. Die geeigneten Verfahrensbedingungen sind oben im Detail beschrieben; es wird auf die obige Offenbarung verwiesen.

Ferner ist ein Kit zur Isolierung und/oder Aufreinigung von Nukleinsäuren, insbesondere von kurzkettigen Nukleinsäuren aus einem nukleinsäurehaltigen Ausgangsmaterial offenbart. Dieses Kit weist Puffer und/oder Reagenzien für die Durchführung des erfindungsgemäßen Verfahrens sowie Instruktionen zur Durchführung des Verfahrens auf. Ein entsprechendes Kit ist insbesondere zur Anreicherung bzw. Aufreinigung von miRNAs aus einer Blutprobe geeignet.

### BEISPIELE

Die vorliegende Erfindung wird nunmehr anhand von Beispielen erläutert. Diese stellen bevorzugte Ausführungsformen der Erfindung dar. Die durchgeführten Versuche erfolgten anhand der nachfolgend beschriebenen Versuchsvorschriften.

Das erfindungsgemäße Protokoll wurde mit einem Im Stand der Technik bekannten Standardprotokoll verglichen, nämlich dem QIAamp Circulating Nucleic Acid Kit (QIAGEN cat. no. 55114). Bei dem QIAamp Circulating Nucleic Acid Kit handelt es sich um eine manuelle Extraktionsprozedur für frei zirkulierende Nukleinsäuren aus Plasma, Serum und anderen zellfreien Körperflüssigkeiten. Der Aufschluss der Probe erfolgt in Gegenwart von chaotropem Salz und einer Proteinase K, die anschließende Bindung der Nukleinsäuren an eine Silicamembran (QIAamp Mini Säule) wird durch ein chaotropes Salz und Isopropanol (ca. 19-20% v/v) vermittelt. Unter diesen Bedingungen können auch sehr kurze RNA-Moleküle, z.B. microRNAs (20-22 nt lang) aufgereinigt werden - allerdings mit einer begrenzten Ausbeute. Die Bindebedingungen des o.g. Standardprotokolls sind für so kurze Nukleinsäuren nicht optimal.

Das o.g. Standard protokoll wurde erfindungsgemäß variiert, in dem zur Bindung der Nukleinsäuren zusätzlich zum Bindepuffer Isopropanol und Detergenz zugesetzt wurde. Die Detailangaben zu den Konzentrationen von Schlüsselkomponenten während der Bindung von Nukleinsäuren an die QIAamp Mini Säule sind in den Tabellen 1 bis 3 angegeben. Als Probenmaterial wurde ein Plasmagemisch mehrerer Blutspender eingesetzt; die Durchführung der Nukleinsäureextraktion erfolgte nach diesem Protokoll.

Es wurden nachfolgende Versuche gemäß dem beschriebenen Versuchsprotokoll durchgeführt:

### I. Beispiel 1

### Aufschluss der Probe

Hierzu wurden 500 µl oder 400 µl QIAGEN Proteinase K in ein 50 ml Röhrchen pipettiert und 5 ml oder 4 ml Plasma hinzugefügt. 3,2 ml ACL Puffer (QIAGEN, ohne Zusatz von Carrier RNA, enthält u.a. ein Chaotrop, ein nicht-ionisches Detergenz sowie Tris-HCL, pH 8.0) wird zugegeben, die Kappe wird verschlossen und durch Puls-vortexen für 30 s gemixt.

Die Probe wurde auf 60°C erhitzt und für 30 min inkubiert. Das Röhrchen wurde kurz geschleudert, um Tropfen aus der Innenseite des Deckels zu beseitigen

### Bindung

Hierzu wurden 7,2 ml des Puffers ACB (QIAGEN, enthält u.a. ein Chaotrop, ein nicht-ionisches Detergenz, Isopropanol sowie Tris-HCL, pH 8.0) zum Lysat gegeben (sowie im Falle der 4ml Proben 6ml, 7ml, 8ml, 10ml und 12ml Isopropanol), der Deckel geschlossen und gründlich durch Puls-Vortexen für 15-30 s gemixt. Die Mischung wird für 5 min auf Eis inkubiert.

Für die Aufreinigung wurde eine Säule (QIAamp Mini Column) eingesetzt. Die Säule wird in einen Vakuumpumpe platziert und ein 20 ml Verlängerungsrohr in die offene Säule platziert. Dabei muss das Verlängerungsrohr fest in die Säule eingesetzt sein, um ein Verlust der Probe zu verhindern. Die aufgeschlossene Probe wird in das Verlängerungsröhrchen der Säule eingeführt und die Vakuumpumpe angeschaltet. Wenn das komplette Lysat durch die Säule gezogen wurde, wird die Vakuumpumpe abgestellt und der Unterdruck auf 0 mbar reduziert. Das Verlängerungsröhrchen wird vorsichtig entfernt.

### DNase Verdau

Die Säule wird in ein 2ml Sammelröhrchen überführt und für 1 min bei 14,000 rpm zentrifugiert. Durch diesen Schritt werden Lysatreste, die ggf. den DNase Verdau behindern könnten entfernt. Pro Probe werden 10µl DNAse Stocklösung zu 70µl Puffer RDD (QIAGEN) gegeben und durch invertieren der Probe gemischt.

Die Säulen werden wieder in ihre ursprünglichen Positionen gebracht. Der DNase I Inkubationsmix (80 µl) wird auf die Silicagel Membran des Säulchens aufgebracht und bei gemäßigten Temperaturen (20-30°C) for 15 min inkubiert.

### Waschen

Zum Waschen werden 600µl Puffer ACW1 (QIAGEN, enthält u.a. ein Chaotrop und Ethanol) auf die Säule gegeben. Der Deckel der Säule wird offen gelassen und die Vakuumpumpe angeschaltet. Nachdem der ganze Puffer ACW1 durch die Säule gelaufen ist, wird die Vakuumpumpe ausgeschaltet und der Druck auf 0 mbar verringert.

750 µl Puffer des Waschpuffers ACW2 (QIAGEN, enthält u.a. ein Chaotrop und Salze) wird auf die Säule gegeben. Der Deckel der Säule wird offen gelassen und die Vakuumpumpe angeschaltet. Nachdem der ganze Puffer ACW2 durch die Säule gelaufen ist, wird die Vakuumpumpe ausgeschaltet und der Druck auf 0 mbar verringert.

Danach werden 750 µl Ethanol (96-100%) auf die Säule gegeben. Der Deckel der Säule wird offen gelassen und die Vakuumpumpe angeschaltet. Nachdem das ganze Ethanol durch die Säule gelaufen ist, wird die Vakuumpumpe ausgeschaltet und der Druck auf 0 mbar verringert.

Der Deckel der Säule wird verschlossen und die Säule in ein sauberes Sammelröhrchen platziert. Danach wird die Säule mit voller Geschwindigkeit (20,000 x g, 14,000 rpm) für 3 min zentrifugiert.

### Elution

Die Säule wird in ein neues 2 ml Sammelröhrchen platziert, der Deckel geöffnet und die Verbindung bei 56°C für 10 min inkubiert, um die Membran komplett zu trocknen.

Die Säule wird in einem sauberen 1.5 ml Elutionsröhrchen platziert und das Sammelröhrchen entfernt. 20-150 µl Elutionspuffer (AVE Puffer, QIAGEN) wird auf das Zentrum der Membran der Säule aufgebracht. Der Deckel wird verschlossen und bei Raumtemperatur für 3 min inkubiert.

Bei voller Geschwindigkeit wird für 1 min zentrifugiert (20,000 x *g*; 14,000 rpm), um die Nukleinsäuren zu eluieren. Im Eluat sind sowohl zirkulierende DNA als auch RNA enthalten.

Tabelle 1 fasst die Bindebedingungen in Beispiel 1 zusammen:

| | Standardprotokoll | + 6 ml IPA | + 7 ml IPA | + 8 ml IPA | + 10 ml IPA | + 12 ml IPA |
|---|---|---|---|---|---|---|
| Gu-Thiocyanat | 2,3 mol/l | 1.6 mol/l | 1,6 mol/l | 1,5 mol/l | 1,4 mol/l | 1,3 mol/l |
| Isopropanol | 19,5% | 42,7% | 45.3% | 47.7% | 51,9% | 55,5% |
| Brij 58 | 10,2% | 7,2% | 6,9% | 6,6 % | 6,1 % | 5,6% |
| Gesamtvolumen | 14,8 ml | 20,8 ml | 21,8 ml | 22,8 ml | 24.8 ml | 26,8 ml |
| Replikate: | 4 repl. | 4 repl. | 4 repl. | 4 repl. | 4 repl. | 4 repl. |

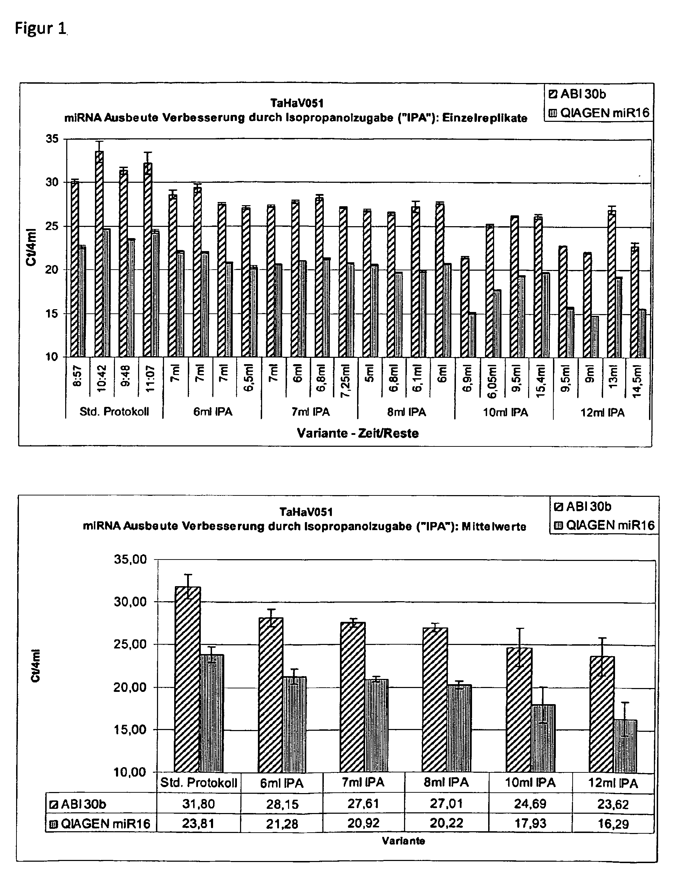

Nach Analyse der miRNA-Ausbeute mittels reverser Transkriptions/real-Time PCR Assays ergab sich Folgendes (siehe auch Fig. 1). Anhand der Ct-Werte (auf ein einheitliches Probenvolumen von 4 ml normalisiert) für die miRNAs 16 und 30b erkennt man, dass mit zunehmender Konzentration an Isopropanol bei der Bindung auch die miRNA-Ausbeute zunimmt. Zudem wurde bereits während der Versuchdurchführung offenbar, dass die Proben nur beim Standardprotokoll komplett über die QIAamp Säulen geladen werden konnten, in allen anderen Fällen verstopften die Säulen so sehr, dass die Lade-Prozedur unterbrochen und restliches Lysat verworfen werden musste. Für das Standardprotokoll sind die Durchlaufzeiten für das Lysat angegeben. In allen anderen Fällen sind die verbleibenden Lysat-Restvolumina dokumentiert (Fig. 1, oberes Grafik).

Offenbar führt die Erhöhung der Isopropanol-Konzentration im Lysat zu Effekten, die das vakuum-basierte Laden des Lysates auf die QIAamp-Säule behindern - möglicherweise durch unspezifische Präzipitation von Plasma-Bestandteilen, die dann als Mikropartikel die Poren der QIAamp-Membran verstopfen. Dieses stellt ein Problem für ein routinemäßiges Abarbeiten des Protokolls dar.

Immerhin konnte die Isopropanolzugabe von 12 ml (ca. 55,5 % v/v im Bindeansatz) eine Verbesserung der miRNA-Ausbeute um 7,5 bis 8,2 Ct's im Vergleich zum Standardprotokoll bewirken - das entspricht einer 180- bis 290-fach erhöhten Ausbeute. Die Ergebnisse zeigt Fig. 1.

### II. Beispiel 2

In einem zweiten Versuch wurden die experimentellen Bedingungen aufgrund der in Versuch 1 erhaltenen Ergebnisse wie folgt modifiziert:
■ Es wurden zur Nukleinsäurebindung auch Isopopanolkonzentrationen unterhalb 42% getestet.
■ Die Menge an Bindepuffer ACB wurde auf 9 ml pro Probe erhöht
■ Bei "6 ml Isopropanol" und "12 ml Isopropanol" wurde auch die Zugabe eines Na-Dodecylsulfat enthaltenden Buffers bspw. ATL (QIAGEN, enthält u.a. Tris pH 7,5 bis 8,5 und SDS) getestet (3 ml Probe und 1 ml Buffer ATL).

Das Protokoll wurde daher wie folgt durchgeführt:

### Aufschluss der Probe

400 µl OIAGEN Proteinase K werden in ein 50 ml Röhrchen pipettiert und 4 ml [3 ml] Plasma hinzugefügt. 3,2 ml ACL Puffer (QIAGEN, ohne Carrier RNA) wird zugegeben sowie im Falle der 3ml Proben zusätzlich 1 ml Puffer ATL, die Kappe wird verschlossen und durch Pulsvortexen für 30 s gemixt.

Die Probe wird auf 60°C erhitzt und für 30 min inkubiert. Das Röhrchen wird kurz geschleudert, um Tropfen aus der Innenseite des Deckels zu beseitigen

### Bindung

Hierzu wurden 7,2 ml des Puffers ACB Puffers (QIAGEN) zum Lysat gegeben (sowie 0ml, 2ml, 4ml und 6ml Isopropanol im Falle der 4 ml Proben und 6ml bzw. 12ml im Falle der 3ml Proben), der Deckel geschlossen und gründlich durch puls-vortexen für 15-30 s gemixt. Die Mischung wird für 5 min auf Eis inkubiert.

Für die Aufreinigung kann eine Säule (QIAamp Mini Column) eingesetzt werden. Die Säule wird in einen VacConnector platziert und ein 20 ml Verlängerungsrohr in die offene Säule platziert. Dabei muss das Verlängerungsrohr fest in die Säule eingesetzt sein, um ein Verlust der Probe zu verhindern. Die aufgeschlossene Probe wird in das Verlängerungsröhrchen der Säule eingeführt und die Vakuumpumpe angeschaltet. Wenn das komplette Lysat durch die Säule gezogen wurde, wird die Vakuumpumpe abgestellt und der Unterdruck auf 0 mbar reduziert. Das Verlängerungsröhrchen wird vorsichtig entfernt.

Die übrigen Schritte (Waschen und Elution) wurden wie in Beispiel 1 beschrieben durchgeführt.

Als Probenmaterial wurde ein Plasmagemisch von verschiedenen Blutspendern eingesetzt - allerdings eine andere Charge, als bei Beispiel 1 verwendet. Die Konzentrationen der wesentlichen Komponenten während der Bindung der Nukleinsäuren an die QIAamp Mini Säule sind in Tabelle 2 dargestellt.

Tabelle 2 fasst die Bindebedingungen in Beispiel 2 zusammen:

| | Standard | + 2 ml IPA | + 4 ml IPA | + 6 ml IPA | +6ml IPA | + 12 ml IPA |
|---|---|---|---|---|---|---|
| Gu-Thiocyanat | 2,3 mol/l | 2,08 mol/l | 1,88 mol/l | 1,71 mol/l | 1,71 mol/l | 1.35mol/l |
| Isopropanol | 19,5 % | 30,1 % | 36,9 % | 42,5 % | 42,5% | 54,5 % |
| Brij 58 | 10,2 % | 9,2 % | 8,3 % | 7.6% | 7,6 % | 6.0 % |
| Na-SDS | -- | -- | -- | -- | 0.13 % | 0.10% |
| Gesamtvolumen | 16.6 ml | 18,6 ml | 20,6 ml | 22,6 ml | 22,6 ml | 28,6 ml |
| | 4 repl. | 4 repl. | 4 repl. | 4 repl. | 4 repl. | 4 repl. |
| | | | | | [1 ml ATL]* | [1 ml ATL]* |

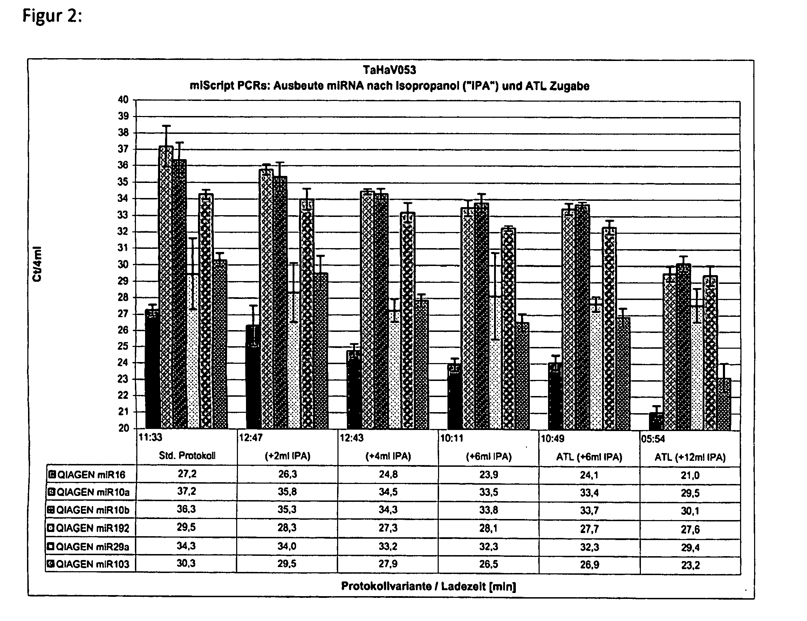

Die Ausbeute der isolierten miRNAs wurde mittels des miScript real-time PCR Assays (QIAGEN) bestimmt. Dabei wurden 6 verschiedene miRNAs untersucht. Es ergab sich folgendes (siehe auch Fig. 2):
■ Wie bereits im Beispiel 1 beobachtet, nimmt die miRNA-Ausbeute aller untersuchten miRNAs mit zunehmender Isopropanolkonzentration bei der Bindung zu; die höchste Ausbeuten finden sich - auf gleiches Probenvolumen bezogen - bei Zugabe von 12 ml Isopropanol (ca. 54,5 % v/v); hier ist die Ausbeute um ca. 2 - 8 Ct-Werte höher (4fach - 250fach) als beim Standardprotokoll.
■ In keinem Fall trat ein Verstopfen der QIAamp-Säulen auf. Überraschenderweise zeigte sich, dass bei 54,5 % Isopropanol und zusätzlich 0,1 % SDS die Zeit für das Passieren der Probe über die QIAamp Mini-Säulen am kürzesten war (ca. 6 Minuten). Bei Einsatz einer Kombination aus hoher Isopropanolkonzentration und Anwesenheit zweier Detergenzien (insbesondere die Kombination ionisch und nicht-ionisch) wurde die Probe hinreichend solubilisiert, um ein Verstopfen der Säule zu verhindern.
■ Der Vergleich der Bedingungen "6 ml Isoprop." und "6 ml Isoprop. + SDS" zeigt, dass die Zugabe von SDS keine signifikanten Einbußen an miRNA-Ausbeute bewirkt.

### III. Beispiel 3

Da aufgrund vorheriger Erfahrungen nicht auszuschließen ist, dass unterschiedliche Plasmaproben unterschiedlich stark dazu neigen, bei der vakuumbasierten Nukleinsäureextraktion die QIAamp-Membranen zu verstopfen, wurde Beispiel 2 mit einem neuen Plasmapool wiederholt: In Beispiel 3 wurden pro Bedingung 6 Replikate untersucht. Das Standardprotokoll des QIAamp Circulating Nucleic Acid Kits wurde mit Varianten verglichen, die bisher die höchsten miRNA-Ausbeuten erzielt hatten. Die Nukleinsäure-Extraktion wurde nach diesem Protokoll durchgeführt:

### Aufschluss der Probe

400 µl QIAGEN Proteinase K werden in ein 50 ml Röhrchen pipettiert und 4 ml [3 ml] Plasma hinzugefügt. 3,2 ml ACL Puffer (QIAGEN) wird zugegeben sowie im Falle der 3ml Proben zusätzlich 1ml Puffer ATL; die Kappe wird verschlossen und durch pulsvortexen für 30 s gemixt.

Die Probe wird auf 60°C erhitzt und für 30 min inkubiert. Das Röhrchen wird kurz geschleudert, um Tropfen aus der Innenseite des Deckels zu beseitigen.

### Bindung

Hierzu wurden 9 ml des Puffers ACB Puffers (QIAGEN) zum Lysat gegeben (6ml Isopropanol im Falle der 4 ml Proben und 6ml bzw. 12ml im Falle der 3ml Proben), der Deckel geschlossen und gründlich durch pulsvortexen für 15-30 s gemixt. Die Mischung wird für 5 min auf Eis inkubiert.

Für die Aufreinigung kann eine Säule (QIAamp Mini Column) eingesetzt werden. Die Säule wird in einen VacConnector platziert und ein 20 ml Verlängerungsrohr in die offene Säule platziert. Dabei muss das Vertängerungsrohr fest in die Säule eingesetzt sein, um ein Verlust der Probe zu verhindern. Die aufgeschlossene Probe wird in das Verlängerungsröhrchen der Säule eingeführt und die Vakuumpumpe angeschaltet. Wenn das komplette Lysat durch die Säule gezogen wurde, wird die Vakuumpumpe abgestellt und der Druck auf 0 mbar reduziert. Das Veriängerungsröhrchen wird vorsichtig entfernt.

Die übrigen Schritte (Waschen und Elution) wurden wie in Versuch 1 beschrieben durchgeführt.

Die Konzentrationen der wesentlichen Komponenten bei der Bindung der Nukleinsäuren sind in Tabelle 3 dargestellt.

**Tabelle 3 fasst die Bindebedingungen in Beispiel 3 zusammen:**

| | Standard | + 6 ml IPA | + 6 ml IPA | + 12 ml IPA |
|---|---|---|---|---|
| Gu-Thiocyanat | 2,3 mol/l | 1,71 mol/l | 1,71 mol/l | 1,35 mol/l |
| Isopropanol | 19.5% | 42.5% | 42.5% | 54,5 % |
| Brij 58 | 10.2 % | 7.6 % | 7,6 % | 6,0 % |
| Na-SDS | -- | -- | 0,13 % | 0,10 % |
| Gesamtvolumen | 14,8 ml | 22.6 ml | 22,6 ml | 28,6 ml |
| | 4repl. | 4repl. | 4repl. | 4repl. |
| | | | [1ml ATL] | [1ml ATL] |

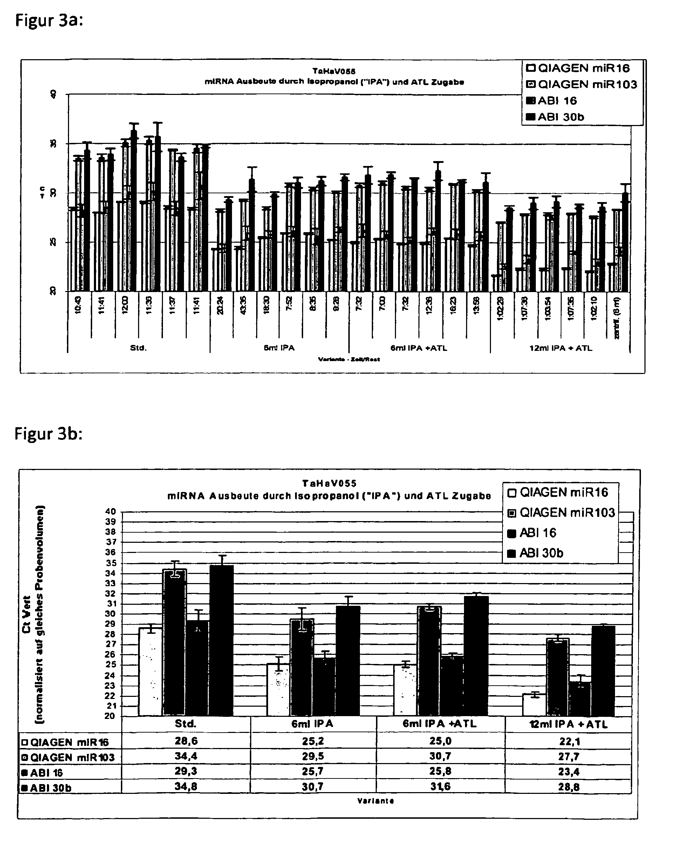

Nach der Nukleinsäureextraktion wurden die isolierten miRNAs mittels QIAGEN miScript und Applied Biosystems TaqMan Assays quantifiziert (siehe auch Fig. 3a/3b).

Bei der Durchführung der miRNA-Extraktion und der miRNA-Quantifizierung zeigte sich folgendes:
■ Bei dieser Plasma-Charge waren die Ladezeiten der QIAamp-Säulen allgemein höher als bei Versuch 2 beobachtet. Wie in Versuch 2, so zeigte sich auch hier überraschenderweise, dass bei 42,5 % Isopropanol bei der Bindung die zusätzliche Anwesenheit von SDS zu einer beschleunigten Passage der Probe führt.
■ Bei 12 ml Isopropanolzugabe (54,5 %) ermögücht die Anwesenheit von SDS hier die komplette Passage der Proben in fast allen Replikaten innerhalb ca. 1 Stunde.
■ Die miRNA-Ausbeute bei 42.5 % Isoprop. liegt ca. 6 Ct-Werte höher als beim Standardprotokoll (60fach - 100fach); bei 54,5% Isoprop. liegt die miRNA-Ausbeute noch ca. 7 - 8fach höher als bei 42,5 % Isopropanol.

Die vorangegangenen Versuche zeigen die Vorteile des erfindungsgemäßen Verfahrens für die Aufreinigung von zirkulierenden miRNAs aus Plasma oder Serum:
Die erfindungsgemäß eingesetzte hohe Isopropanolkonzentration ist von Vorteil für die miRNA-Ausbeute. Insbesondere ist es vorteilhaft, Isopropanol anstelle von Ethanol zu verwenden, da bei Einsatz von Ethanol höhere Konzentrationen (60% (v/v)) eingesetzt werden müssen. Letzteres führt bei der Aufreinigung von miRNA aus Plasma/Serum zur starken Erhöhung des gesamten Probenvolumens. Dies ist möglichst zu vermeiden, da das Probenvolumen für die Extraktion frei zirkulierender Nukleinsäuren ohnehin vorzugsweise hoch ist, um eine für diagnostische Anwendungen ausreichende Ausbeute zu erzielen. Ein sehr hohes Gesamtvolumen bei der Nukleinsäure-Extraktion ist ungünstig für die Abarbeitung des Protokolls, da sich das Vakuum-basierte Laden der Säulen allgemein verlängert. Hohe Volumina sind auch ungünstig für die automatisierte Abarbeitung des Extraktionsprotokolls.
Der Einsatz einer hohen Isopropanol(Alkohol-)Konzentration senkt zwar die Viskosität der Probe, führt aber auch zur unspezifischen Präzipitation von Komponenten des Probenlysates, die dann bei Vakuumprozessierung in Form kleiner Partikel die Bindemembran verstopfen können. Ein gleichzeitiger Einsatz zweier Detergenzien (z.B. Brij 58 und SDS) führt überraschenderweise zur Verringerung der Verstopfungsneigung (durch spezifische bessere Solubilisierung von Isopropanol-induzierten Präzipitaten) und damit zur deutlich vereinfachten Durchführung der vakuum-basierten Extraktion. Dieses wird durch Anwendung nur eines Detergenz (z.B. Brij 58) allein nicht erreicht. Damit können die vorteilhafte Wirkung der hohen Isopropanolkonzentration auf die miRNA-Ausbeute genutzt, die Nachteile aber zugleich vermieden werden. Das in Versuch 3 dargestellte Protokoll (1,3-2,5) mol/l Gu-Thiocyanat. 42-60% Isopropanol bei 1, vorzugsweise 4 -6% Brij 58 und 0,1-1,0% SDS) kann insbesondere zur miRNA-Extraktion für diagnostische Anwendungen eingesetzt werden.

## Patentansprüche

1. Verfahren zur Isolierung und/oder Aufreinigung von Nukleinsäuren, insbesondere kurzkettigen Nukleinsäuren aus einem nukleinsäurehaltigen Ausgangsmaterial, **gekennzeichnet durch** die folgenden Verfahrensschritte:
(a) Bindung der Nukleinsäuren an ein nukleinsäurebindendes Trägermaterial, indem man das Ausgangsmaterial in Gegenwart wenigstens einer chaotropen Verbindung, wenigstens eines anionischen und wenigstens eines nicht-ionischen Detergenz und wenigstens eines Alkohols ausgewählt aus Isopropanol und Ethanol, mit dem nukleinsäurebindenden Trägermaterial in Kontakt bringt, wobei der Alkohol in einer Konzentration ≥ 40 % (v/v) vorliegt und wobei das nukleinsäurebindende Trägermaterial eine nukleinsäurebindende Membran, eine lose Schüttung eines porösen oder nicht porösen Trägermaterials, ein nukleinsäurebindender Filter oder eine nukleinsäurebindende Filterschicht ist;
(b) Elution der gebundenen Nukleinsäuren von dem nukleinsäurebindenden Trägermaterial.

2. Verfahren nach Anspruch 1, **dadurch gekennzeichnet, dass** Isopropanol als Alkohol eingesetzt wird.

3. Verfahren nach Anspruch 2, **gekennzeichnet durch** ein oder mehrere der folgenden Merkmale:
a) die Detergenzien sind Tenside;
b) das anionische Detergenz wird in einer Menge von wenigstens ≥ 0,05% (v/v) eingesetzt;
c) das nicht-ionische Detergenz wird in einer Menge von wenigstens ≥ 1 % (w/v), vorzugsweise ≥ 4% (w/v) eingesetzt;
d) das anionische Detergenz ist ein Sulfat oder Sulfonat eines Fettalkohols, vorzugsweise ausgewählt aus der Gruppe Natriumdodecylsulfat, Natriumdodecylsulfonat, Dodecylbenzolsulfonsäure, N-Lauroylsarcosin, Natriumcholat, am meisten bevorzugt Natriumdodecylsulfat;
e) das nicht- ionische Detergenz ist eine Polyoxyethylen-Verbindung, insbesondere ein Polyoxyethylen-Alkylether, vorzugsweise ausgewählt aus der Gruppe Brij 58, Brij 30, Brij 35, Brij 56, Brij 72, Brij 90, Tween 20, Tween 80, Triton X-100, Triton X-114, Triton X-450, Igepal CA-630, Nonidet P-40, vorzugsweise Brij 58; und/oder
f) als Detergenzien werden mindestens Brij 58 und SDS eingesetzt.

4. Verfahren nach einem oder mehreren der Ansprüche 1 bis 3, wobei das Trägermaterial eines oder mehrere der nachfolgenden Merkmale aufweist:
a) die Dicke der Membran, Filter oder Filterschicht liegt in einem Bereich von 0,5 bis 3mm oder 0,7 bis 1,3mm;
b) das Flächengewicht liegt bei 50-400g/m2 oder 100 bis 180 g/m2; und/oder
c) die Partikelretention liegt bei 0,05µm bis 100µm oder 0,1 bis 20µm.

5. Verfahren nach einem oder mehreren der Ansprüche 1 bis 4, **dadurch gekennzeichnet, dass** die chaotrope Verbindung in einer Konzentration von wenigstens ≥ 1,0 mol/l eingesetzt wird.

6. Verfahren nach einem oder mehreren der Ansprüche 1 bis 5, **dadurch gekennzeichnet, dass** der Alkohol in einer Menge von ≥ 42,5 % (v/v), vorzugsweise ≥ 50% (v/v) eingesetzt wird.

7. Verfahren nach einem oder mehreren der Ansprüche 1 bis 6, **dadurch gekennzeichnet, dass** das nukleinsäurehaltige Ausgangsmaterial eines oder mehrere der nachfolgenden Merkmale aufweist:
a) es ist eine zellfreie Probe und/oder eine entsprechend aufbereitete Probe;
b) es handelt sich um Blut, Plasma oder Serum; und/oder
c) das nukleinsäurehaltige Ausgangsmaterial liegt in einem Volumen von ≥ 1ml, vorzugweise ≥ 3 ml vor.

8. Verfahren nach einem oder mehreren der Ansprüche 1 bis 7, **dadurch gekennzeichnet, dass** das Elutionsvolumen ≥ 10 bis s 200 µl, vorzugsweise ≥ 10 bis s 150 µl beträgt.

9. Verfahren nach einem oder mehreren der Ansprüche 1 bis 8, **dadurch gekennzeichnet, dass** die zu isolierenden Nukleinsäuren eines oder mehrere der nachfolgenden Merkmale aufweisen:
a) sie weisen eine Länge von ≤ 500nt, ≤ 400nt und/oder ≤ 300nt und/oder ≤ 100nt auf;
b) es handelt sich um extrazelluläre Nukleinsäuren;
c) es handelt sich um nicht-proteinkodierende RNAs; und/oder
d) es handelt sich um RNAi auslösende Nukleinsäuren.

10. Verfahren nach einem oder mehreren der Ansprüche 1 bis 9 zur Isolierung von miRNA.

11. Verfahren nach einem oder mehreren der Ansprüche 1 bis 9 zur Isolierung und/oder Anreicherung von RNAi auslösenden Nukleinsäuren, insbesondere miRNAs aus einer Blutprobe, insbesondere aus Blutplasma oder -serum.

12. Verfahren nach einem oder mehreren der Ansprüche 1 bis 11, **gekennzeichnet durch** eines oder mehrere der nachfolgenden Merkmale:
a) die zu isolierenden Nukleinsäuren, insbesondere Nukleinsäuren wie in Anspruch 9 definiert, liegen in dem nukleinsäurehaltigen Ausgangsmaterial in einem Gemisch mit anderen Nukleinsäuren vor;
b) die zu isolierenden Nukleinsäuren wie in Anspruch 9 definiert, liegen nach der Aufreinigung und/oder Isolierung in einem Gemisch mit anderen Nukleinsäuren vor; und/oder
c) die Nukleinsäuren wie in Anspruch 9 definiert, die nach der Aufreinigung/Isolierung in einem Gemisch mit anderen Nukleinsäuren vorliegen können, werden im Anschluss an die Aufreinigung/Isolierung weiterverarbeitet, insbesondere prozessiert, modifiziert, amplifiziert und/oder analysiert.

13. Verfahren nach einem oder mehreren der Ansprüche 1 bis 12 zur Anwendung in der Diagnostik.

14. Verfahren nach einem oder mehreren der Ansprüche 1 bis 13, **dadurch gekennzeichnet, dass** das Trägermaterial ausgewählt ist aus der Gruppe der Silikahaltigen Materialien, Silikagel, Siliziumdioxid, Kieselgel und Glas.

15. Verfahren nach einem oder mehreren der Ansprüche 1 bis 14, **dadurch gekennzeichnet, dass** die isolierten Nukleinsäuren analysiert werden.

## Claims

1. A method of isolating and/or purifying nucleic acids, in particular short-chain nucleic acids from a nucleic acid-containing starting material **characterized by** the following steps:
(a) binding of the nucleic acids to a nucleic acid-binding support material by contacting the starting material with said nucleic acid-binding support material in the presence of at least one chaotropic compound, at least one anionic and at least one non-ionic detergent and at least one alcohol selected from isopropanol and ethanol, wherein the alcohol is present in a concentration ≥ 40% (v/v) and wherein said nucleic acid-binding support material is a nucleic acid-binding membrane, a bulk of a porous or nonporous support material, a nucleic acid-binding filter or a nucleic acid-binding filter layer;
(b) elution of the bound nucleic acids from the nucleic acid-binding support material.

2. The method according to claim 1, wherein isopropanol is used as alcohol.

3. The method according to claim 2, **characterized by** one or more of the following features:
a) the detergents are surfactants;
b) the anionic detergent is used in an amount of at least ≥ 0.05% (v/v);
c) the non-ionic detergent is used in an amount of at least ≥ 1% (w/v), preferably ≥ 4% (w/v);
d) the anionic detergent is a sulfate or sulfonate of a fatty alcohol, preferably selected from the group of sodium dodecyl sulfate, sodium dodecyl sulfonate, dodecylbenzenesulfonic acid, N-lauroylsarcosine, sodium cholate, most preferably sodium dodecyl sulfate;
e) the non-ionic detergent is a polyoxyethylene compound, in particular a polyoxyethylene alkyl ether, preferably selected from the group of Brij 58, Brij 30, Brij 35, Brij 56, Brij 72, Brij 90, Tween 20, Tween 80, Triton X-100; Triton X-114; Triton X-450, Igepal CA-630, Nonidet P-40, preferably Brij 58; and/or
f) as detergents at least Brij 58 and SDS are used.

4. The method according to one or more of claims 1 to 3, wherein the support material has one or more of the following features:
a) the thickness of the membrane, filter or filter layer is in a range of 0.5 to 3 mm or 0.7 to 1.3 mm;
b) the mass per unit area is 50-400 g/m² or 100 to 180 g/m²; and/or
c) the particle retention is in a range of 0.05 µm to 100 µm or 0.1 to 20 µm.

5. The method according to one or more of claims 1 to 4, wherein the chaotropic compound is used in a concentration of at least ≥ 1.0 mol/l.

6. The method according to one or more of claims 1 to 5, wherein the alcohol is used in an amount of ≥ 42.5% (v/v), preferably ≥ 50% (v/v).

7. The method according to one or more of claims 1 to 6, wherein the nucleic acid-containing starting material has one or more of the following characteristics:
a) it is a cell-free sample and/or a sample prepared accordingly;
b) it is blood, plasma or serum; and/or
c) the nucleic acid-containing starting material is present in a volume of ≥ 1 ml, preferably ≥ 3 ml.

8. The method according to one or more of claims 1 to 7, wherein the elution volume is ≥ 10 to ≤ 200 µl, preferably ≥ 10 to ≥ 150 µl.

9. The method according to one or more of claims 1 to 8, wherein the nucleic acids to be isolated have one or more of the following characteristics:
a) they have a length of ≤ 500 nt, ≤ 400 nt and/or ≤ 300 nt and/or ≤ 100 nt in length;
b) they are extracellular nucleic acids;
c) they are non-protein-coding RNAs; and/or
d) they are RNAi-inducing nucleic acids.

10. The method according to one or more of claims 1 to 9 for isolation of miRNA.

11. The method according to one or more of claims 1 to 9 for isolation and/or enrichment of RNAi-inducing nucleic acids, in particular miRNAs from a blood sample, in particular from blood plasma or serum.

12. The method according to one or more of claims 1 to 11, **characterized by** one or more of the following features:
a) the nucleic acids to be isolated, in particular nucleic acids as defined in claim 9, are present in the nucleic acid-containing starting material in a mixture with other nucleic acids;
b) the nucleic acids to be isolated as defined in claim 9 are present in a mixture with other nucleic acids after purification and/or isolation; and/or
c) the nucleic acids as defined in claim 9, which after purification/isolation can be present in a mixture with other nucleic acids are subsequent to the purification/isolation further processed in particular processed, modified, amplified and/or analyzed after said purification/isolation.

13. The method according to one or more of claims 1 to 12 for use in diagnostics.

14. The method according to one or more of claims 1 to 13, wherein the support material is selected from the group of silica-containing materials, silicon dioxide gel, silicon dioxide, silica gel and glass.

15. The method according to one or more of claims 1 to 14, wherein the isolated nucleic acids are analyzed.

## Revendications

1. Procédé d'isolation et/ou de purification d'acides nucléiques, notamment d'acides nucléiques à chaîne courte à partir d'un matériau de départ contenant des acides nucléiques, **caractérisé par** les étapes de procédé suivantes :
(a) fixation des acides nucléiques au matériau support de fixation à des acides nucléiques en ce que l'on met en contact le matériau de départ en présence d'au moins un composé chaotropique, d'au moins un détergent anionique et d'au moins un détergent non ionique et d'au moins un alcool sélectionné parmi l'isopropanol et l'éthanol, avec le matériau support de fixation à des acides nucléiques, dans lequel l'alcool est présent à une concentration ≥ 40 % (v/v) et dans lequel le matériau support de fixation à des acides nucléiques est une membrane de fixation à des acides nucléiques, un versement en vrac d'un matériau support poreux ou non poreux, un filtre de fixation à des acides nucléiques ou une couche de filtre de fixation à des acides nucléiques;
(b) élution des acides nucléiques fixés du matériau support de fixation à des acides nucléiques.

2. Procédé selon la revendication 1, **caractérisé en ce que** l'isopropanol est utilisé comme alcool.

3. Procédé selon la revendication 2, **caractérisé par** une ou plusieurs des caractéristiques suivantes:
a) les détergents sont des tensioactifs;
b) le détergent anionique est utilisé en une quantité d'au moins ≥ 0,05 % (v/v);
c) le détergent non ionique est utilisé en une quantité d'au moins ≥ 1 % (p/v), de préférence ≥ 4 % (p/v);
d) le détergent anionique est un sulfate ou sulfonate d'un alcool gras, de préférence sélectionné parmi le groupe dodécylsulfate sodique, dodécylsulfonate sodique, acide dodécylbenzènesulfonique, N-lauroylsarcosine, cholate sodique, de manière préférée entre toutes dodécylsulfate sodique;
e) le détergent non ionique est un composé de polyoxyéthylène, notamment un éther alkylique de polyoxyéthylène, de préférence sélectionné parmi le groupe Brij 58, Brij 30, Brij 35, Brij 56, Brij 72, Brij 90, Tween 20, Tween 80, Triton X-100, Triton X-114, Triton X-450, Igepal CA-630, Nonidet P-40, de préférence Brij 58; et/ou
f) au moins Brij 58 et SDS sont utilisés comme détergents.

4. Procédé selon une ou plusieurs des revendications 1 à 3, dans lequel le matériau support présente une ou plusieurs des caractéristiques suivantes:
a) l'épaisseur de la membrane, du filtre ou de la couche de filtre se situe dans une plage de 0,5 à 3 mm ou 0,7 à 1,3 mm;
b) le poids superficiel se situe à 50 à 400 g/m² ou 100 à 180 g/m²; et/ou
c) la rétention particulaire se situe à 0,05 µm à 100 µm ou 0,1 à 20 µm.

5. Procédé selon une ou plusieurs des revendications 1 à 4, **caractérisé en ce que** le composé chaotropique est utilisé à une concentration d'au moins ≥ 1,0 mol/l.

6. Procédé selon une ou plusieurs des revendications 1 à 5, **caractérisé en ce que** l'alcool est utilisé en une quantité de ≥ 42,5 % (v/v), de préférence ≥ 50 % (v/v).

7. Procédé selon une ou plusieurs des revendications 1 à 6, **caractérisé en ce que** le matériau de départ contenant des acides nucléiques présente une ou plusieurs des caractéristiques suivantes:
a) il s'agit d'un échantillon exempt de cellules et/ou d'un échantillon préparé en conséquence;
b) il s'agit de sang, plasma ou sérum; et/ou
c) le matériau de départ contenant des acides nucléiques est présent à un volume de ≥ 1 ml, de préférence ≥ 3 ml.

8. Procédé selon une ou plusieurs des revendications 1 à 7, **caractérisé en ce que** le volume d'élution est ≥ 10 à ≤ 200 µl, de préférence ≥ 10 à ≤ 150 µl.

9. Procédé selon une ou plusieurs des revendications 1 à 8, **caractérisé en ce que** les acides nucléiques à isoler présentent une ou plusieurs des caractéristiques suivantes:
a) ils présentent une longueur de ≤ 500 nt, ≤ 400 nt et/ou ≤ 300 nt et/ou ≤ 100 nt;
b) il s'agit d'acides nucléiques extracellulaires;
c) il s'agit d'ARN ne codant pas pour des protéines ; et/ou
d) il s'agit d'acides nucléiques déclenchant de l'ARNi.

10. Procédé selon une ou plusieurs des revendications 1 à 9 pour l'isolation d'ARNmi.

11. Procédé selon une ou plusieurs des revendications 1 à 9 pour l'isolation et/ou l'enrichissement d'acides nucléiques déclenchant de l'ARNi, notamment d'ARNmi à partir d'un échantillon sanguin, notamment de plasma ou sérum sanguin.

12. Procédé selon une ou plusieurs des revendications 1 à 11, **caractérisé par** une ou plusieurs des caractéristiques suivantes:
a) les acides nucléiques à isoler, notamment des acides nucléiques comme définis à la revendication 9, sont présents dans le matériau de départ contenant des acides nucléiques dans un mélange avec d'autres acides nucléiques ;
b) les acides nucléiques à isoler comme définis à la revendication 9 sont présents après la purification et/ou l'isolation dans un mélange avec d'autres acides nucléiques ; et/ou
c) les acides nucléiques comme définis à la revendication 9 qui peuvent être présents après la purification/l'isolation dans un mélange avec d'autres acides nucléiques sont transformés davantage, notamment traités, modifiés, amplifiés et/ou analysés suite à la purification/l'isolation.

13. Procédé selon une ou plusieurs des revendications 1 à 12 pour l'application dans le diagnostic.

14. Procédé selon une ou plusieurs des revendications 1 à 13, **caractérisé en ce que** le matériau support est sélectionné parmi le groupe des matériaux contenant de la silice, du gel de silice, du dioxyde de silicium, du gel siliceux et du verre.

15. Procédé selon une ou plusieurs des revendications 1 à 14, **caractérisé en ce que** les acides nucléiques isolés sont analysés.
